# EUROPEAN PATENT APPLICATION

(11) **EP 2 085 058 A2**
(43) Date of publication of application: **05.08.2009**
(21) Application number: 09250223.6
(22) Date of filing: 28.01.2009
(51) Int. Cl.: A61F 5/00

(54) **Sensor trigger**

(30) Priority: 29.01.2008 US 21814
(71) Applicant: Ethicon Endo-Surgery, Inc., Cincinnati, OH 45242 (US)
(72) Inventor: Ortiz, Mark S., Milford, Ohio 45150 (US); Dlugos, Daniel F., Jr., Middletown, Ohio 45044 (US); Marcotte, Amy L., Mason, Ohio 45040 (US); Albrecht, Thomas E., Cincinnati, Ohio 45242 (US); Stokes, Michael J., Cincinnati, Ohio 45244 (US); Plescia, David N., Cincinnati, Ohio 45227 (US); Yates, David C., West Chester, Ohio 45069 (US); Doll, Kevin, Mason, Ohio 45040 (US)
(74) Representative: Tunstall, Christopher Stephen

(57) **Abstract**

Methods and devices for effecting a gastric restriction system are disclosed. In one exemplary embodiment, a restriction system for forming a restriction in a patient is provided and can include an implantable restriction device and at least one implantable sensor that is in communication with the restriction device. In general, the implantable restriction device can be adjustable and can be configured to form a restriction in a patient. The implantable sensor(s) can be defaulted to a dormant power usage mode and can have a triggering mechanism that is configured to place the sensor(s) in a use configuration upon the occurrence of a triggering event.

## Description

### FIELD OF THE INVENTION

The present invention relates to methods and devices for effecting a gastric restriction system, in particular, triggering a sensor of a gastric restriction system.

### BACKGROUND OF THE INVENTION

Obesity is becoming a growing concern, particularly in the United States, as the number of obese people continues to increase, and more is learned about the negative health effects of obesity. Morbid obesity, in which a person is 100 pounds or more over ideal body weight, in particular poses significant risks for severe health problems. Accordingly, a great deal of attention is being focused on treating obese patients. One method of treating morbid obesity has been to place a restriction device, such as an elongated band, about the upper portion of the stomach. Gastric bands have typically comprised a fluid-filled elastomeric balloon with fixed endpoints that encircles the stomach just inferior to the esophageal-gastric junction to form a small gastric pouch above the band and a reduced stoma opening in the stomach. When fluid is infused into the balloon, the band expands against the stomach creating a food intake restriction or stoma in the stomach. To decrease this restriction, fluid is removed from the band. The effect of the band is to reduce the available stomach volume and thus the amount of food that can be consumed before becoming "full."

Food restriction devices have also comprised mechanically adjusted bands that similarly encircle the upper portion of the stomach. These bands include any number of resilient materials or gearing devices, as well as drive members, for adjusting the bands. Additionally, gastric bands have been developed that include both hydraulic and mechanical drive elements. An example of such an adjustable gastric band is disclosed in U.S. Pat. No. 6,067,991, entitled "Mechanical Food Intake Restriction Device" which issued on May 30, 2000, and is incorporated herein by reference. It is also known to restrict the available food volume in the stomach cavity by implanting an inflatable elastomeric balloon within the stomach cavity itself. The balloon is filled with a fluid to expand against the stomach walls and, thereby, decrease the available food volume within the stomach.

With each of the above-described food restriction devices, safe, effective treatment requires that the device be regularly monitored and adjusted to vary the degree of restriction applied to the stomach. With banding devices, the gastric pouch above the band will substantially increase in size following the initial implantation. Accordingly, the stoma opening in the stomach must initially be made large enough to enable the patient to receive adequate nutrition while the stomach adapts to the banding device. As the gastric pouch increases in size, the band may be adjusted to vary the stoma size. In addition, it is desirable to vary the stoma size in order to accommodate changes in the patient's body or treatment regime, or in a more urgent case, to relieve an obstruction or severe esophageal dilatation. Traditionally, adjusting a hydraulic gastric band required a scheduled clinician visit during which a Huber needle and syringe were used to penetrate the patient's skin and add or remove fluid from the balloon via the injection port. More recently, implantable pumps have been developed which enable non-invasive adjustments of the band. An external programmer communicates with the implanted pump using telemetry to control the pump. During a scheduled visit, a physician places a handheld portion of the programmer near the gastric implant and transmits power and command signals to the implant. The implant in turn adjusts the fluid levels in the band and transmits a response command to the programmer.

During these gastric band adjustments, it has been difficult to determine how the adjustment is proceeding, and whether the adjustment will have the intended effect. In an attempt to determine the efficacy of an adjustment, some physicians have utilized fluoroscopy with a Barium swallow as the adjustment is being performed. However, fluoroscopy is both expensive and undesirable due to the radiation doses incurred by both the physician and patient. Other physicians have instructed the patient to drink a glass of water during or after the adjustment to determine whether the water can pass through the adjusted stoma. This method, however, only assures that the patient is not obstructing, and does not provide any information about the efficacy of the adjustment. Oftentimes, a physician may simply adopt a "try as you go" method based upon their prior experience, and the results of an adjustment may not be discovered until hours or days later, when the patient experiences a complete obstruction to the stomach cavity, or the band induces erosion of the stomach tissue due to excessive interface pressures against the band.

It is often desirable to collect data concerning the operation of the restriction system as well as concerning the physiological characteristics of the patient. Thus, some restriction systems are equipped with a variety of sensors that can be configured to collect and transmit data that is useful for adjustment, diagnostic, monitoring, and other purposes. However, the operating power requirements of these sensors often make it prohibitive to maintain constant operation on an internalpower source and there is thus a need to conserve power usage until required.

Accordingly, methods and devices are provided for use with a gastric restriction device, and in particular for operating an internal sensor only when necessary.

### SUMMARY OF THE INVENTION

The present invention generally provides devices and methods for effecting a gastric restriction system. In one exemplary embodiment, a restriction system for forming a restriction in a patient is provided and can include an implantable restriction device and an implantable sensor that is in communication with the restriction device. In general, the implantable restriction device can be adjustable and can be configured to form a restriction in a patient. The implantable sensor(s) can be defaulted to a dormant power usage mode and can have a triggering mechanism that is configured to place the sensor(s) in a use configuration upon the occurrence of a triggering event. In one exemplary embodiment, the implantable sensor(s) can be completely shut-off in the dormant power usage mode. In another embodiment, the dormant power usage mode can correspond to a low operating frequency, such as an operating frequency of less than or equal to 1 Hz. In general, the use configuration can have an operating frequency of about 2 to 20 Hz.

The restriction system can also include an implantable port that is in fluid communication with the implantable restriction device and is configured to receive fluid from a fluid source that is external to the patient. In one embodiment, the implantable sensor can be integrated with the implantable port. The triggering mechanism can be formed on the implantable sensor, the implantable port, or at another location within the restriction system.

Various configurations are available for the triggering mechanism and the triggering event. Such configurations range from mechanisms that trigger the implantable sensor upon the detection of a change in a physiological characteristic of the patient to mechanisms that can be manually activated by the patient or physician.

In one exemplary embodiment, the triggering mechanism can include at least one gastric pH sensor and the triggering event is a change in gastric pH of a selected magnitude detected by the gastric pH sensor. In another embodiment, the triggering mechanism can include at least one pressure sensor and the triggering event is a change in pressure of a selected magnitude detected by the pressure sensor. In yet another embodiment, the trigging mechanism includes a flexible membrane and the triggering event is an increase in pressure within the restriction device that is effective to deflect the flexible membrane. The flexible membrane can be at least partially conductive and deflecting the membrane can be effective to complete an electrical circuit to energize the sensor and place it in the use configuration.

Another embodiment of a triggering mechanism includes an actuator and the triggering event includes actuation of the actuator. The triggering mechanism can also include a magnetic sensor and the triggering event includes generating at least one magnetic field thereby engaging the triggering mechanism. In another embodiment, the triggering mechanism can include a photoreceptor and the triggering event includes subjecting the photoreceptor to a light source using an external device. Another embodiment of a triggering mechanism can include an accelerometer and the triggering event includes transmitting vibratory energy within a selected frequency range transdermally to the accelerometer with an external actuator. Yet another embodiment of a triggering mechanism can include at least one temperature sensor and the triggering event includes a temperature change of a selected magnitude or frequency detected by the temperature sensor. The triggering mechanism can also include a timer programmed to place the sensor in the use configuration at pre-determined intervals. In one embodiment, the timer can be pre-programmed prior to implantation. The timer can also be configured such that it can be adjusted by an external device after implantation.

Methods for effecting a gastric restriction system are also provided. In one exemplary embodiment, a method of effecting gastric restriction includes providing an implantable restriction system, such as the one described above, triggering a sensor(s) of the restriction system in response to a selected stimulus to energize the sensor(s) from a dormant power usage mode to a use mode, collecting data related to the operation of a restriction device of the system via the sensor(s) when the sensor(s) is in the use mode, and transmitting the data collected by the sensor(s) to an external device when the sensor(s) is in the use mode. The method can also include adjusting the restriction device in response to the data collected and transmitted by the sensor(s).

The collowing is a non-exhaustive list of embodiments of the invention that are or may be claimed.
1. A restriction system for forming a restriction in a patient, comprising: an implantable restriction device, the restriction device being adjustable and configured to form a restriction in a patient; and an implantable sensor in communication with the restriction device, the sensor being defaulted to a dormant power usage mode and having a triggering mechanism configured to place the sensor in a use configuration upon the occurrence of a triggering event.
2. The restriction system of embodiment 1, further comprising an implantable port in fluid communication with the implantable restriction device and configured to receive fluid from a fluid source external to the patient.
3. The restriction system of embodiment 2, wherein the implantable sensor is integrated with the port.
4. The restriction system of embodiment 2, wherein the triggering mechanism is formed on either the implantable sensor or the implantable port.
5. The restriction system of embodiment 1, wherein the dormant power usage mode has an operating frequency less than or equal to about 1 Hz.
6. The restriction system of embodiment 1, wherein the use configuration has an operating frequency in the range of about 2 to 20 Hz.
7. The restriction system of embodiment 1, wherein the triggering mechanism includes at least one gastric pH sensor and the triggering event is a change in gastric pH of a selected magnitude detected by the gastric pH sensor.
8. The restriction system of embodiment 1, wherein the triggering mechanism includes at least one pressure sensor and the triggering event is a change in pressure of a selected magnitude detected by the pressure sensor.
9. The restriction system of embodiment 1, wherein the trigging mechanism includes a flexible membrane and the triggering event is an increase in pressure within the restriction device that is effective to deflect the flexible membrane.
10. The restriction system of embodiment 9, wherein the flexible membrane is at least partially conductive and deflecting the membrane is effective to complete an electrical circuit to energize the sensor and place it in the use configuration.
11. The restriction system of embodiment 1, wherein the triggering mechanism is an actuator and the triggering event includes actuation of the actuator.
12. The restriction system of embodiment 1, wherein the triggering mechanism includes a magnetic sensor and the triggering event includes generating at least one magnetic field thereby engaging the triggering mechanism.
13. The restriction system of embodiment 1, wherein the triggering mechanism includes a photoreceptor and the triggering event includes subjecting the photoreceptor to a light source using an external device.
14. The restriction system of embodiment 1, wherein the triggering mechanism includes an accelerometer and the triggering event includes transmitting vibratory energy within a selected frequency range transdermally to the accelerometer with an external actuator.
15. The restriction system of embodiment 1, wherein the triggering mechanism includes at least one temperature sensor and the triggering event includes a temperature change of a selected magnitude or frequency detected by the temperature sensor.
16. The restriction system of embodiment 1, wherein the triggering mechanism includes a timer programmed to place the sensor in the use configuration at pre-determined intervals.
17. The restriction system of embodiment 16, wherein the timer is pre-programmed prior to implantation.
18. The restriction system of embodiment 16, wherein the timer can be adjusted by an external device.
19. A method of effecting gastric restriction, comprising: providing an implantable restriction system for forming a restriction in a patient, the system including an adjustable restriction device configured to form a restriction in a patient, and a sensor in communication with the restriction device and being selectively configured between a dormant power usage mode and a use mode; triggering the sensor in response to a selected stimulus to energize the sensor from the dormant power usage mode to the use mode; collecting data related to the operation of the adjustable restriction device via the sensor when the sensor is in the use mode; and transmitting the data collected by the sensor to an external device when the sensor is in the use mode.
20. The method of embodiment 19, further comprising adjusting the restriction device in response to the data collected and transmitted by the sensor.
21. The method of embodiment 19, wherein the dormant power usage mode has an operating frequency less than or equal to about 1 Hz.
22. The method of embodiment 19, wherein the use configuration has an operating frequency in the range of about 2 to 20 Hz.
23. The method of embodiment 19, wherein the selected stimulus is selected from the group including a gastric pH change of a selected magnitude, a pressure change of a selected magnitude within the restriction system, and a temperature change of a selected magnitude or frequency within the restriction system.
24. The method of embodiment 19, wherein triggering the sensor includes subjecting a magnetic sensor disposed in the restriction system to at least one magnetic field thereby engaging the triggering mechanism.
25. The method of embodiment 19, wherein triggering the sensor includes subjecting a photoreceptor disposed in the restriction system to a light source using an external device.
26. The method of embodiment 19, wherein triggering the sensor includes actuating an actuator operatively associated with the implantable sensor.
27. The method of embodiment 19, wherein triggering the sensor includes transmitting vibratory energy within a selected frequency range transdermally to an accelerometer disposed in the restriction system with an external actuator.
28. The method of embodiment 19, wherein triggering the sensor includes automatically energizing the sensor to the use mode at pre-determined time intervals.
29. A method of energizing an implantable sensor, comprising: providing an implantable sensor in communication with an adjustable gastric restriction device, the sensor being selectively configured between a dormant power usage mode and a use mode for collecting and transmitting data related to the operation of the adjustable gastric restriction device; and triggering the sensor in response to a selected stimulus to energize the sensor from the dormant power usage mode to the use mode.
30. The method of embodiment 29, wherein the selected stimulus is selected from the group including a gastric pH change of a selected magnitude, a pressure change of a selected magnitude within the restriction system, and a temperature change of a selected magnitude or frequency within the restriction system.
31. The method of embodiment 29, wherein triggering the sensor includes subjecting a magnetic sensor disposed in the restriction system to at least one magnetic field thereby engaging the triggering mechanism.
32. The method of embodiment 29, wherein triggering the sensor includes subjecting a photoreceptor disposed in the restriction system to a light source using an external device.
33. The method of embodiment 29, wherein triggering the sensor includes actuating an actuator operatively associated with the restriction system.
34. The method of embodiment 29, wherein triggering the sensor includes transmitting vibratory energy within a selected frequency range transdermally to an accelerometer disposed in the restriction system with an external actuator.
35. The method of embodiment 29, wherein triggering the sensor includes automatically energizing the sensor to the use mode at pre-determined time intervals.

### BRIEF DESCRIPTION OF THE DRAWINGS

The invention will be more fully understood from the following detailed description taken in conjunction with the accompanying drawings, in which:

FIG. 1A is a perspective view of one embodiment of a food intake restriction system;

FIG. 1B is perspective view of one embodiment of a restriction system;

FIG. 2A is a perspective view of the gastric band of the restriction system shown in FIG. 1B;

FIG. 2B is a perspective view of the gastric band shown in FIG. 2A as applied to the gastro-esophageal junction of a patient;

FIG. 3 is a perspective view of the fluid injection port of the restriction system shown in FIG. 1B;

FIG. 4 is a perspective view of another embodiment of a restriction system;

FIG. 5 is a block diagram of one embodiment of a pressure management system for use in conjunction with the restriction system shown in FIG. 4;

FIG. 6A is a block diagram of one embodiment of a pressure management system having a triggering mechanism that includes a gastric pH sensor;

FIG. 6B is a block diagram of another embodiment of a pressure management system having a triggering mechanism that includes a gastric pH sensor;

FIG. 6C is a block diagram of another embodiment of a pressure management system having a triggering mechanism that includes a gastric pH sensor;

FIG. 6D is a schematic of an esophagogastric pH probe assembly used in a study to measure pH at various parts of the esophagus and stomach;

FIG. 6E shows the results from the study that measured the esophagogastric pH with a probe assembly similar to the one shown in FIG. 6D;

FIG. 7A is a block diagram of one embodiment of a pressure management system having a triggering mechanism that includes a temperature sensor;

FIG. 7B is a block diagram of another embodiment of a pressure management system having a triggering mechanism that includes a temperature sensor;

FIG. 7C is a block diagram of another embodiment of a pressure management system having a triggering mechanism that includes a temperature sensor;

FIG. 8A is a block diagram of one embodiment of a pressure management system having a triggering mechanism that includes a timer;

FIG. 8B shows the effect of a programmed timer, such as the one shown in FIG. 8A, on a pressure sensor of a restriction system;

FIG. 9A is a perspective view of one embodiment of a triggering mechanism of a restriction system as applied to a pressure sensor;

FIG. 9B is a perspective cross-sectional view of the triggering mechanism shown in FIG. 9A;

FIG. 9C is an enlarged view of the triggering mechanism shown in FIG. 9B;

FIG. 10A is a perspective view of one embodiment of a triggering mechanism of a restriction system wherein the triggering mechanism includes an actuator;

FIG. 10B is a perspective view of another embodiment of a triggering mechanism of a restriction system wherein the triggering mechanism includes an actuator;

FIG. 10C is a perspective view of another embodiment of a triggering mechanism of a restriction system wherein the triggering mechanism includes an actuator;

FIG. 11 is a perspective view of one embodiment of a triggering mechanism of a restriction system wherein the triggering mechanism includes a photoreceptor;

FIG. 12 is a perspective view of one embodiment of a triggering mechanism of a restriction system wherein the triggering mechanism includes an accelerometer; and

FIG. 13 is a perspective view of one embodiment of a triggering mechanism of a restriction system wherein the triggering mechanism is actuated from within the stomach.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention generally provides methods and devices for effecting a gastric restriction system. In one exemplary embodiment, a restriction system for forming a restriction in a patient is provided and can include an implantable restriction device and at least one implantable sensor that is in communication with the restriction device. In general, the implantable restriction device can be adjustable and can be configured to form a restriction in a patient. The implantable sensor can be defaulted to a dormant power usage mode and can have a triggering mechanism that is configured to place the sensor in a use configuration upon the occurrence of a triggering event. The triggering mechanism can thus facilitate activation of the implantable sensor from the dormant power usage mode to the use mode in response to a selected stimulus. Various configurations are available for the triggering mechanism and the triggering event. Such configurations range from mechanisms that trigger the implantable sensor upon the detection of a change in a physiological characteristic of the patient to mechanisms that can be manually activated by the patient or physician. Upon the occurrence of the triggering event and activation of the implantable sensor to the use mode, the sensor can then collect data related to the operation of the restriction device and transmit the collected data to an external device.

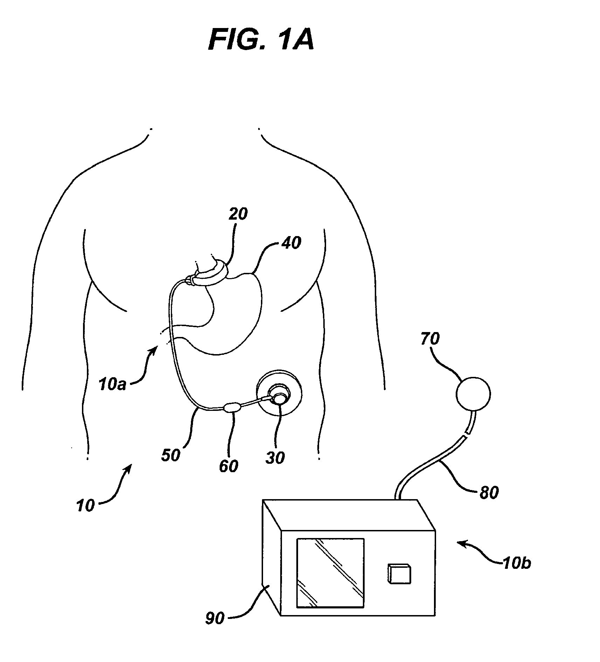

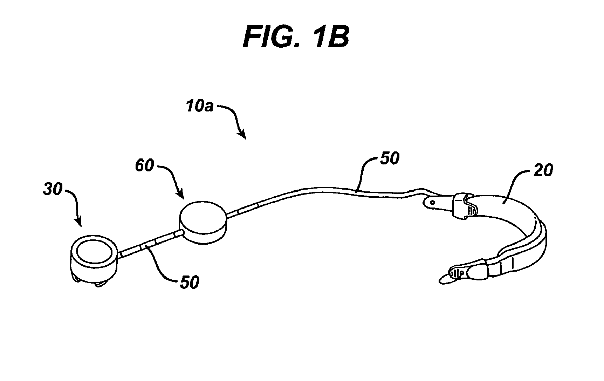

While the present invention can be used with a variety of restriction systems known in the art, FIGS. 1A and 1B illustrate one exemplary embodiment of a food intake restriction system 10. As shown, the system 10 generally includes an implantable portion 10a and an external portion 10b. The implantable portion 10a includes an adjustable gastric band 20 that is configured to be positioned around the upper portion of a patient's stomach 40, and an injection port 30 that is fluidly coupled to the adjustable gastric band 20, e.g., via a catheter 50. The injection port 30 is adapted to allow fluid to be introduced into and removed from the gastric band 20 to thereby adjust the size of the band, and thus the pressure applied to the stomach. The injection port 30 can thus be implanted at a location within the body that is accessible through the tissue. Typically, injection ports are positioned in the lateral subcostal region of the patient's abdomen under the skin and layers of fatty tissue. Surgeons also typically implant injection ports on the sternum of the patient. The internal portion can also include at least one sensor or measuring device that can be adapted to monitor the operation of the restriction system. The sensor can have a variety of configurations and can be adapted to measure any number of operational parameters of the system and/or physiological characteristics of the patient. In one exemplary embodiment, shown in FIG. 1A, the sensor takes the form of a pressure measuring device that is in fluid communication with the closed fluid circuit in the implantable portion 10a such that the pressure measuring device can measure the fluid pressure of the closed fluid circuit. While the pressure measuring device can have various configurations and it can be positioned anywhere along the internal portion 10a, including within the injection port 30, in the illustrated embodiment the pressure measuring device is in the form of a pressure sensor that is disposed within a sensor housing 60 positioned adjacent to the injection port 30. The catheter 50 can include a first portion that is coupled between the gastric band 20 and the pressure measuring port 60, and a second portion that is coupled between the pressure sensor housing 60 and the injection port 30. As further shown in FIG. 1A, the external portion 10b generally includes a pressure reading device 70 that is configured to be positioned on the skin surface above the pressure sensor housing 60 (which can be implanted beneath thick tissue, e.g., over 10 cm thick) to non-invasively communicate with the pressure measuring port 60 and thereby obtain pressure measurements. The pressure reading device 70 can optionally be electrically coupled (in this embodiment via an electrical cable assembly 80) to a control box 90 that can display the pressure measurements, or other data obtained from the pressure reading device 70.

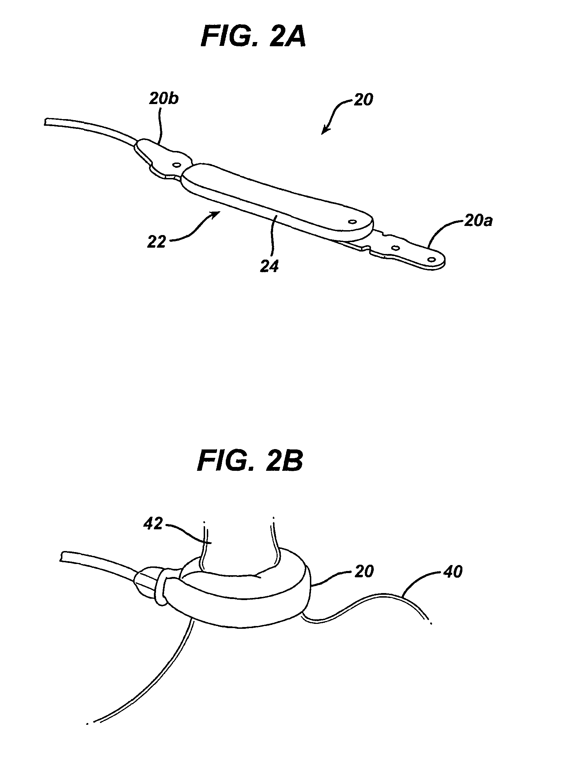

FIG. 2A shows the gastric band 20 in more detail. While the gastric band 20 can have a variety of configurations, and various gastric bands currently known in the art can be used with the present invention, in the illustrated embodiment the gastric band 20 has a generally elongate shape with a support structure 22 having first and second opposite ends 20a, 20b that can be secured to each other. Various mating techniques can be used to secure the ends 20a, 20b to one another. In the illustrated embodiment, the ends 20a, 20b are in the form of straps that mate together, with one laying on top of the other. The gastric band 20 can also includes a variable volume member, such as an inflatable balloon 24, that is disposed or formed on one side of the support structure 22, and that is configured to be positioned adjacent to tissue. The balloon 24 can expand or contract against the outer wall of the stomach to form an adjustable stoma for controllably restricting food intake into the stomach. A person skilled in the art will appreciate that the gastric band can have a variety of other configurations such as a non-hydraulic based restrictive device, moreover the various methods and devices disclosed herein have equally applicability to other types of implantable bands. For example, bands are used for the treatment of fecal incontinence, as described in U.S. Pat. No. 6,461,292 which is hereby incorporated herein by reference. Bands can also be used to treat urinary incontinence, as described in U.S. Patent Application 2003/0105385 which is hereby incorporated herein by reference. Bands can also be used to treat heartburn and/or acid reflux, as disclosed in U.S. Pat. No. 6,470,892 which is hereby incorporated herein by reference. Bands can also be used to treat impotence, as described in U.S. Patent Application 2003/0114729 which is hereby incorporated herein by reference.

FIG. 2B shows the adjustable gastric band 20 applied about the gastro-esophageal junction of a patient. As shown, the band 20 at least substantially encloses the upper portion of the stomach 40 near the junction with the esophagus 42. After the band 20 is implanted, preferably in the deflated configuration wherein the band 20 contains little or no fluid, the band 20 can be inflated, e.g., using saline, to decrease the size of the stoma opening. A person skilled in the art will appreciate that various techniques, including mechanical and electrical techniques, can be used to adjust the band.

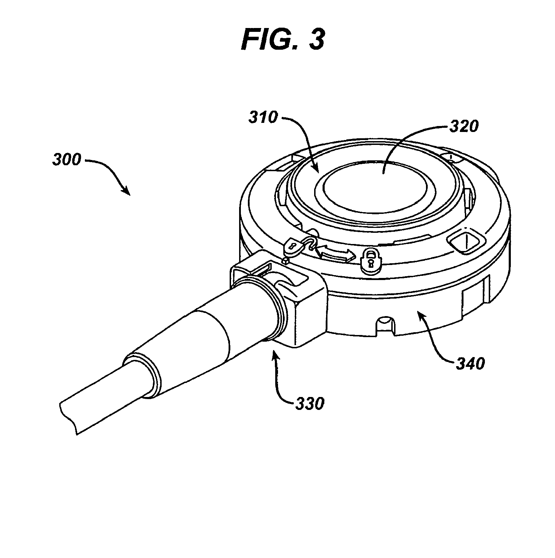

The fluid injection port can also have a variety of configurations. In the embodiment shown in FIG. 3, the injection port 300 has a generally cylindrical housing 340 with a distal or bottom surface and a perimeter wall extending proximally from the bottom surface and defining a proximal opening 310. The proximal opening 310 can include a needle-penetrable septum 320 extending there across and providing access to a fluid reservoir formed within the housing 340. The septum 320 can be placed in a proximal enough position such that the depth of the reservoir is sufficient enough to expose the open tip of a needle, such as a Huber needle, so that fluid transfer can take place. The septum 320 is typically arranged so that it will self seal after being punctured by a needle and the needle is withdrawn. As further shown in FIG. 3, the port 300 can further include a catheter tube connection member 330 that is in fluid communication with the reservoir and that is configured to couple to a catheter. A person skilled in the art will appreciate that the housing 340 can be made from any number of materials, including stainless steel, titanium, or polymeric materials, and the septum can likewise be made from any number of materials, including silicone.

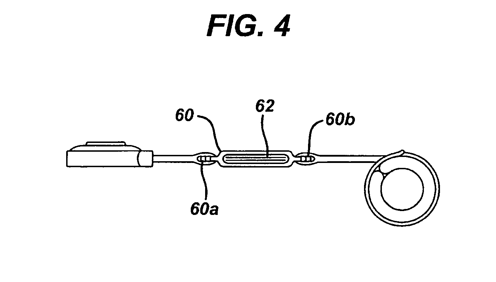

As indicated above, the system can also include one or more sensors for monitoring the operation of the gastric restriction system. The sensor(s) can be configured to measure various operational parameters of the system including, but not limited to, the pressure, pH, diameter, and temperature within the system. In one exemplary embodiment, the system can include a sensor in the form of a pressure measuring device that is in communication with the closed fluid circuit and that is configured to measure the fluid pressure, which corresponds to the amount of restriction applied by the adjustable gastric band to the patient's stomach. Measuring the fluid pressure enables a physician to evaluate the restriction created by a band adjustment. In the illustrated embodiment, the pressure measuring device is in the form of a pressure sensor that is disposed within a sensor housing 60. The pressure measuring device can, however, be disposed anywhere within the closed hydraulic circuit of the implantable portion. For example, in one embodiment, the implantable sensor can be integrated with the port. Additional exemplary locations and configurations are disclosed in more detail in commonly-owned U.S. Publication No. 2006/0211913 entitled "Non-Invasive Pressure Measurement In a Fluid Adjustable Restrictive Device, filed on March 7, 2006, and hereby incorporated by reference in its entirety. In general, as shown in FIG. 4, the illustrated housing 60 includes an inlet 60a and an outlet 60b that are in fluid communication with the fluid in the system. The sensor 62 is disposed within the housing 60 and is configured to respond to fluid pressure changes within the hydraulic circuit and convert the pressure changes into a usable form of data. While not shown, the pressure sensing system can also include a microcontroller, a TET/telemetry coil, and a capacitor. Optionally, the pressure sensing system can further comprise a temperature sensor (not shown). Microcontroller, TET/telemetry coil, and capacitor can be in communication via a circuit board (not shown) or via any other suitable component(s). It will also be appreciated that TET/telemetry coil and capacitor may collectively form a tuned tank circuit for receiving power from external portion, and transmitting the pressure measurement to the pressure reading device.

Various pressure sensors known in the art can be used, such as a wireless pressure sensor provided by CardioMEMS, Inc. of Atlanta, Georgia, though a suitable MEMS pressure sensor may be obtained from any other source, including but not limited to Integrated Sensing Systems (ISSYS), and Remon Medical. One exemplary MEMS pressure sensor is described in U.S. Patent No. 6,855,115, the disclosure of which is incorporated by reference herein for illustrative purposes only. It will also be appreciated that suitable pressure sensors may include, but are not limited to, capacitive, piezoresistive, silicon strain gauge, or ultrasonic (acoustic) pressure sensors, as well as various other devices capable of measuring pressure.

The pressure reading device 70 can also have a variety of configurations, and one exemplary pressure reading device is disclosed in more detail in commonly-owned U.S. Publication No. 2006/189888, entitled "Device for non-invasive measurement of fluid pressure in an adjustable restriction device," filed on February 24, 2005, and U.S. Publication No. 2006/0199997A1, entitled "Monitoring of a food intake restriction device," filed on April 6, 2006, which are hereby incorporated by reference in their entirety. In general, the pressure reading device 70 can non-invasively measure the pressure of the fluid within implanted portion even when the injection port 30 or pressure measuring device 60 is implanted beneath thick (at least over 10 centimeters) subcutaneous fat tissue. The physician may hold pressure-reading device 70 against the patient's skin near the location of sensor and observe the pressure reading on a display on the control box 90. The pressure reading device 70 can also be removably attached to the patient, such as during a prolonged examination, using straps, adhesives, and other well-known methods. The pressure reading device 70 can operate through conventional cloth or paper surgical drapes, and can also include a disposal cover (not shown) that may be replaced for each patient.

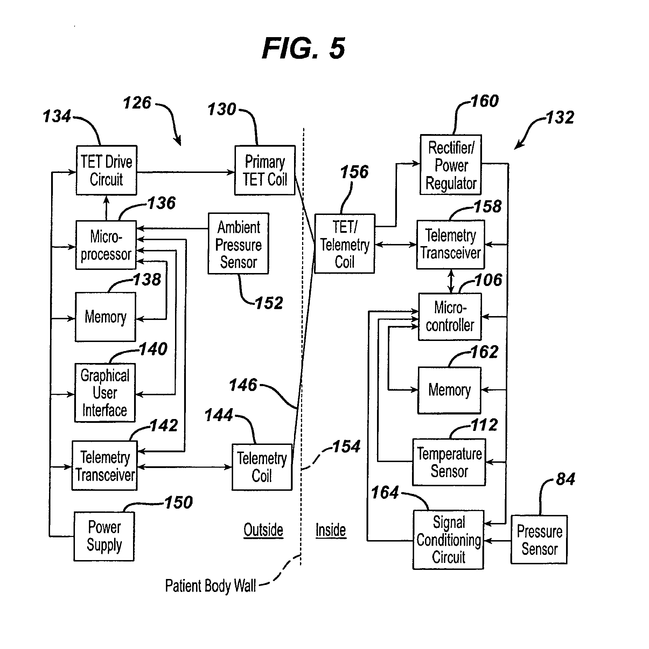

FIG. 5 is a block diagram of one exemplary embodiment of a pressure measurement system for use in conjunction with the pressure sensor described above. As shown in FIG. 5, an external control module 126 of the system includes a primary TET coil 130 for transmitting a power signal to the internal control module, indicated generally as 132. Primary TET coil 130 is located in pressure reading device 60 shown in FIG. 1. A TET drive circuit 134 controls the application of a power signal to primary TET coil 130. TET drive circuit 134 is controlled by a microprocessor 136 having an associated memory 138. A graphical user interface 140 is connected to microprocessor 136 for controlling the data shown on display 66. External control module 126 also includes a primary telemetry transceiver 142 for transmitting interrogation commands to and receiving response data, including fluid pressure readings, from implant control module 132. Primary transceiver 142 is electrically connected to microprocessor 136 for inputting and receiving command and data signals. Primary transceiver 142 resonates at a selected RF communication frequency to generate a downlink alternating magnetic field 146 that transmits command data to implant control module 132. A power supply 150 supplies energy to external control module 126 in order to power system 30. An ambient pressure sensor 152 is connected to microprocessor 136. Microprocessor 136 uses the signal from ambient pressure sensor 152 to adjust the pressure reading for variations in atmospheric pressure due to, for example, variations in barometric conditions or altitude, in order to increase the accuracy of the pressure measurement.

FIG. 5 also illustrates internal control module 132 that can be implanted beneath the patient's skin 154. Internal control module 132 is located within the housing of the injection port. As shown in FIG. 5, a secondary TET/telemetry coil 156 in internal control module 132 receives power and communication signals from external control module 126. Coil 156 forms a tuned tank circuit that is inductively coupled with either primary TET coil 130 to power the implant, or primary telemetry coil 144 to receive and transmit data. A telemetry transceiver 158 controls data exchange with coil 156. Additionally, internal control module 132 includes a rectifier/power regulator 160, microcontroller 106 described above, a memory 162 associated with the microcontroller, temperature sensor 112, pressure sensor 84 and a signal conditioning circuit 164 for amplifying the signal from the pressure sensor. Internal control module 132 transmits the temperature adjusted pressure measurement from pressure sensor 84 to external control module 126. In external module 126, the received pressure measurement signal is adjusted for changes in ambient pressure and shown on a display.

As indicated above, the sensor element can be defaulted to a dormant power usage mode. A dormant power mode is intended to conserve usable power from an internal battery, capacitor, or other type of power storage. For example, in one exemplary embodiment, the implant can be partially dormant such that only the sensor interrogation portion of the circuit is powered continuously and another portion of the circuit, such as the telemetry circuit, is in a dormant or sleep mode. Such a configuration can reduce the power usage of the implant thereby reducing the required power capacity of an internal battery of the restriction system. In general, it is not necessary for the sensor element to be continuously operating at full capacity. Thus, the sensor can be defaulted to the dormant power usage mode and energized when sensor readings are desirable, for example, when a patient is consuming food. In the default or dormant power usage mode the sensor is in a non-operational state (i.e., it is not actively sensing, collecting, or transmitting data related to an operating parameter of the system or physiological characteristic of a patient). In one exemplary embodiment, the implantable sensor can be completely shut-off in the dormant power usage mode. In another embodiment, the dormant power usage mode can correspond to a low operating frequency, such as an operating frequency of less than or equal to about 1 Hz. At the low operating frequency, some very low power functions can remain active such as a timer and some microcontroller functions. In general, the use configuration can have an operating frequency in the range of about 2 to 20 Hz. It shall be understood that higher or lower sampling frequencies can be used to conserve more or less power depending upon operational need of the system. Nyquist frequency or Nyquist rate principles can be used to determine the cut-off frequency of a given sampling system. The sampling frequency is intended to allow a sufficient sampling interval to detect a change in the physiologic feedback from the sensors. For example, in one exemplary embodiment, a pressure sensor can detect a higher pressure swallowing event at the lower sampling rate which can then signal the system to increase the sampling rate to capture and record data from the swallowing pulses.

A triggering mechanism can be associated with the sensor and can be configured to place the sensor in a use configuration upon the occurrence of a triggering event. For example, the triggering mechanism can bring the system out of the dormant mode for some time to determine if the event or subsequent events require further action by the system. The triggering mechanism can be disposed at a variety of locations within the restriction system. For example, in one exemplary embodiment, the triggering mechanism can be disposed on or integrally formed with the implantable sensor. In another embodiment, the triggering mechanism can be disposed on or integrally formed with the implantable port. Various configurations are available for the triggering mechanism and the triggering event. Such configurations range from triggering mechanisms that automatically energize the implantable sensor in response to a change in a physiological characteristic of the patient to triggering mechanisms that can be manually activated by the patient or physician. Various exemplary embodiments of triggering mechanisms and corresponding triggering events are described below.

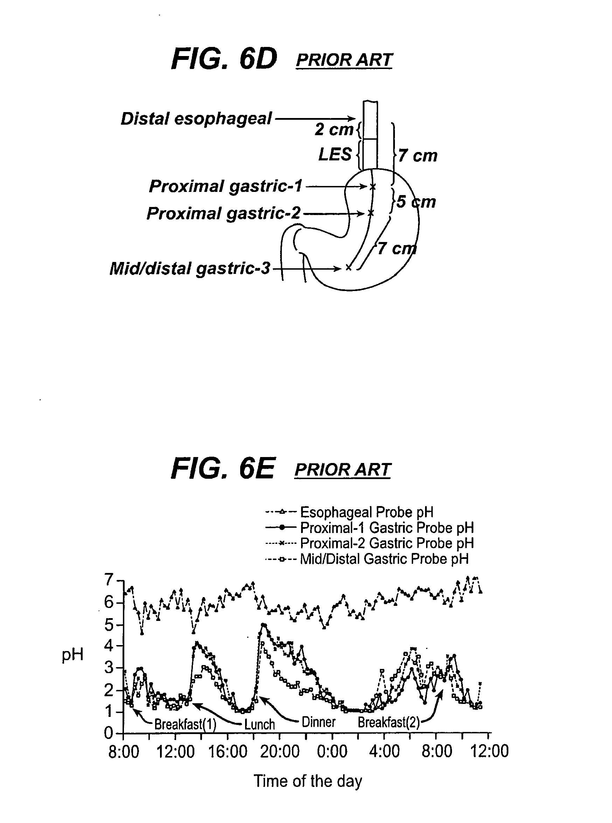

In one exemplary embodiment, the triggering mechanism can include a gastric pH sensor and the triggering event can be a change in gastric pH of a selected magnitude. Variations in gastric pH can indicate whether or not there is food present in the stomach. The relationship between gastric pH levels and food consumption is explained in detail in "Regional Postprandial Differences in pH Within the Stomach and Gastroesophageal Junction," Digestive Diseases and Sciences, Vol. 50, No. 12 (December 2005), pgs. 2276-2285. In general, gastric pH is low in an empty stomach. Upon eating, especially foods that contain protein, gastric pH becomes more basic (i.e., the pH value increases) due to buffering by the food. The increase in pH occurs even though the stomach is actively secreting acid. Once the buffering capacity of the food is exceeded, the gastric pH returns to a low value. FIGS. 6D and 6E, which are reproduced from the above referenced article, illustrate the change in gastric pH over time. FIG. 6D is a schematic of an esophagogastric pH probe assembly that includes four pH probes disposed at various points in the esophagus and stomach. The pH probes, indicated by the X, are disposed in the distal esophagus, the proximal stomach, and the mid/distal stomach. FIG. 6E shows the results from a study that measured the esophagogastric pH with a similar probe assembly over a 27 hour period. As illustrated in FIG. 6E, the gastric pH increases after each meal and returns to the baseline pH sometime thereafter.

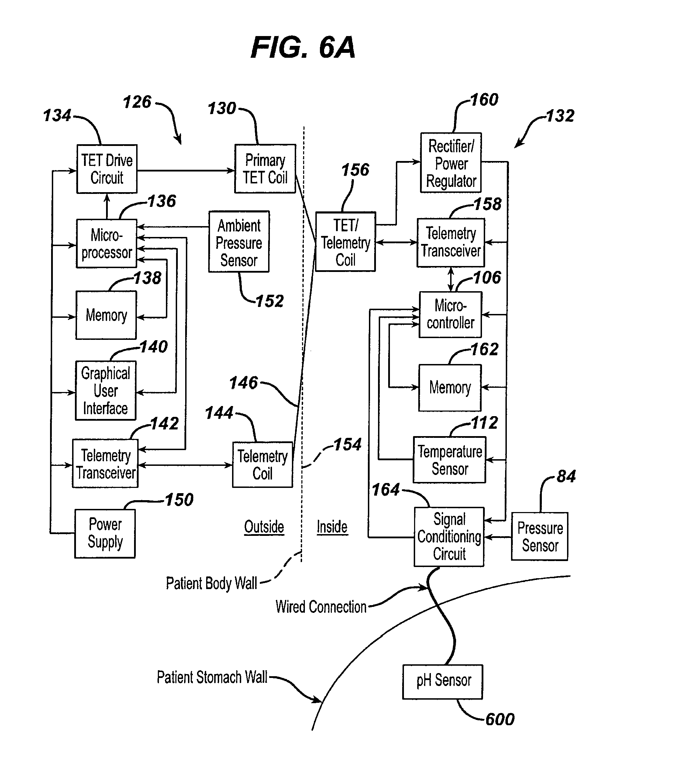

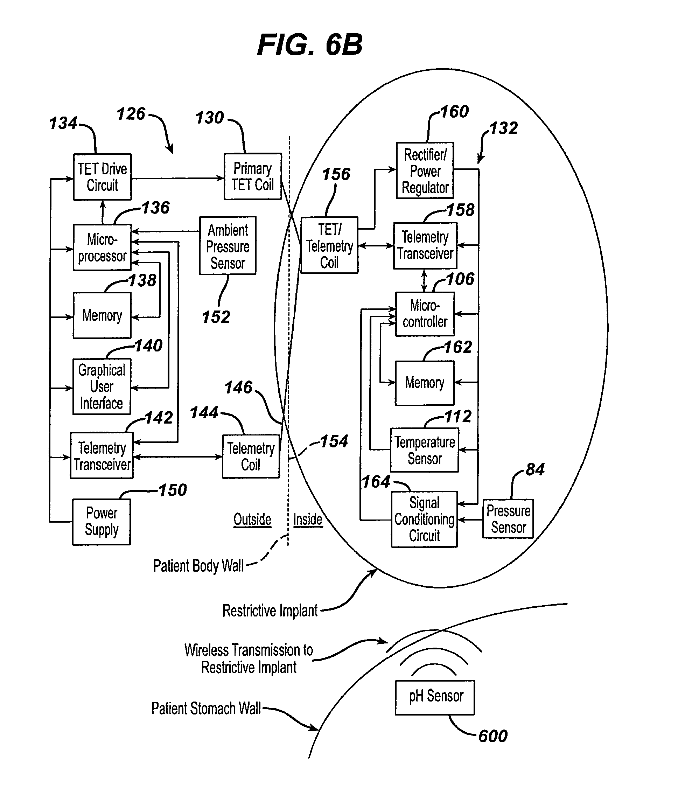

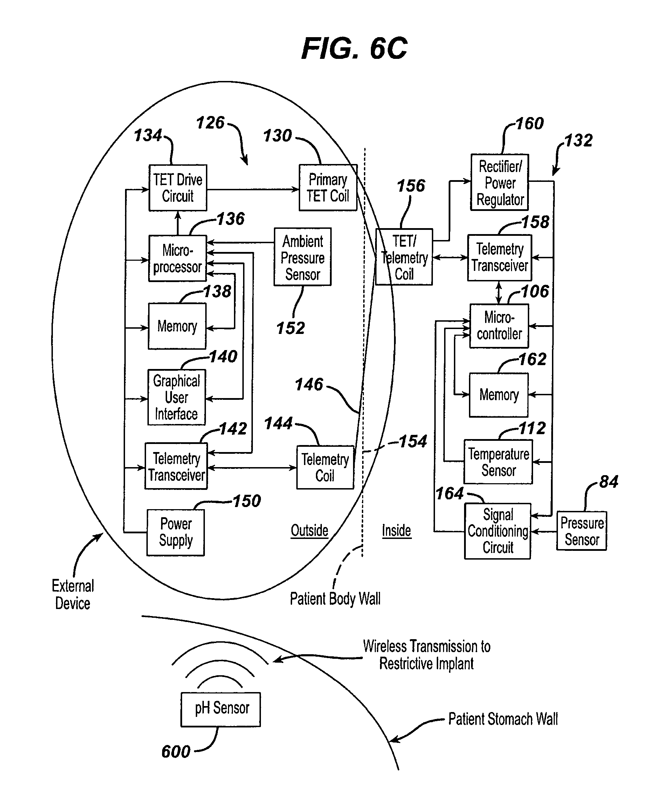

FIG. 6A illustrates one exemplary embodiment of a triggering mechanism that includes a gastric pH sensor 600 for use with an implantable sensor of a restriction system. For purposes of illustration, the triggering mechanism is shown in association with the pressure measurement system described above with respect to FIG. 5. However, one skilled in the art that will appreciate that the triggering mechanism can be used in conjunction with any type of sensor and its application should not be limited exclusively to pressure sensors or pressure measurement systems. As shown in FIG. 6A, the triggering mechanism takes the form of a gastric pH sensor 600 that is operatively associated with the pressure sensor 84. As discussed above, the gastric pH sensors can be positioned at numerous locations in the patient including, for example, the distal esophagus, the proximal stomach, and the mid/distal stomach. One skilled in the art will appreciate that one or more gastric pH sensors can be disposed at a variety of locations within the esophagus and stomach. The triggering event can generally be a change in gastric pH of a selected magnitude (e.g., |X - Y| pH). Alternatively, the triggering event can be a change in gastric pH that results in a pH range that is less than 7 pH. Thus, by way of example, in one exemplary embodiment, a change in gastric pH detected by the gastric pH sensor that is less than 7 pH can be effective to trigger the implantable sensor, thereby energizing the implantable sensor from the dormant power usage mode to a use configuration. One skilled in the art will appreciate that various techniques can be used to energize the system. In general, the pH sensor can send a signal to the implantable sensor to energize. In one exemplary embodiment, the signal can be sent by means of a direct connection between the pH sensor and the implantable sensor via a wired connection through the stomach (FIG. 6A). In another embodiment, the pH sensor can wirelessly communicate with the implantable sensor (FIG. 6B). In yet another embodiment, the pH sensor can transmit a signal to an external collection device that communicates the pH levels to the implantable sensor (FIG. 6C).

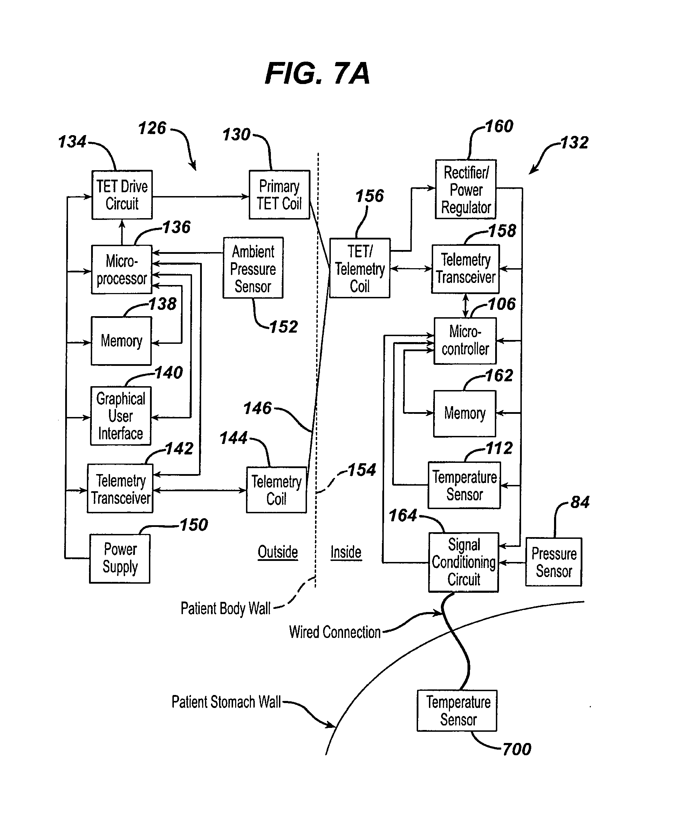

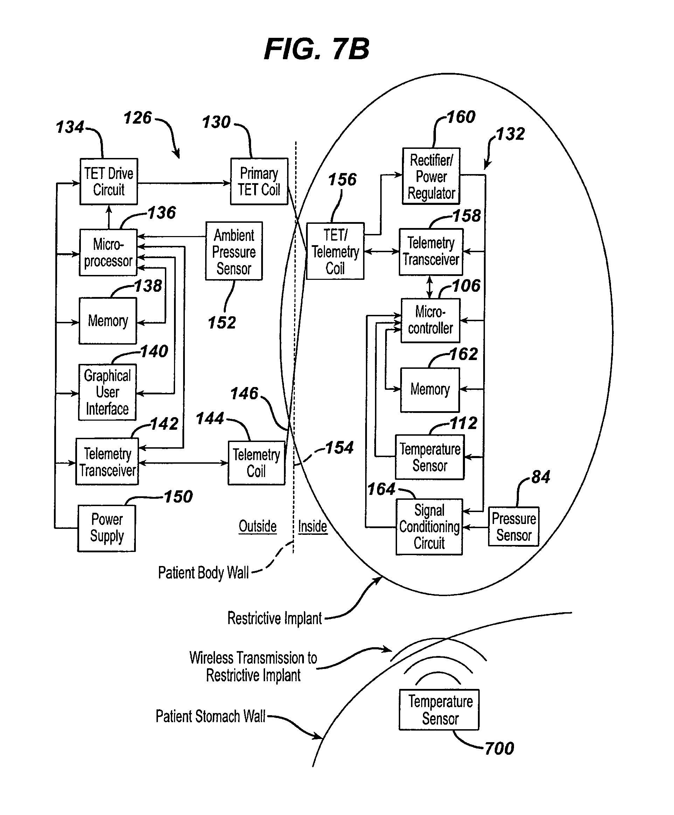

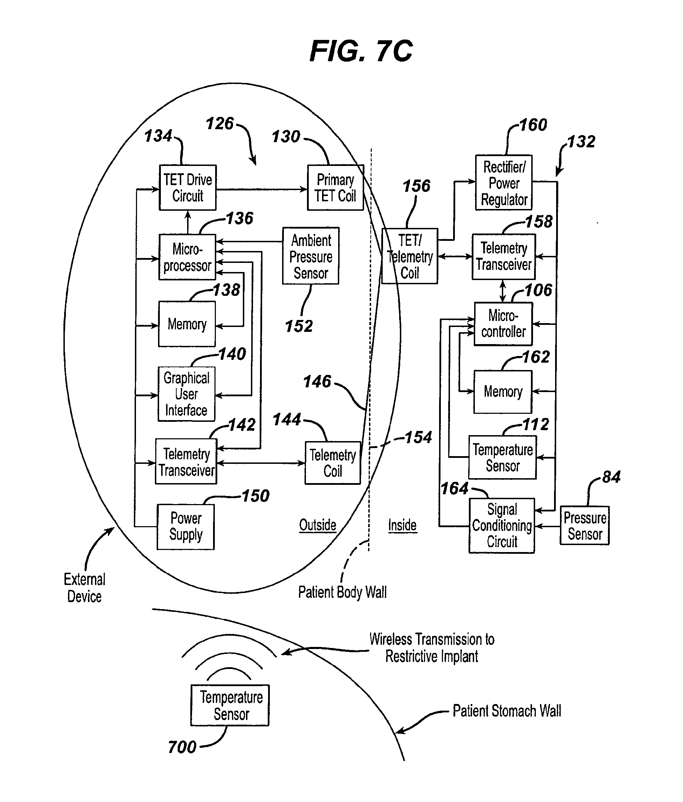

In another exemplary embodiment, the triggering mechanism can include a temperature sensor and the triggering event can be a change in temperature of a selected magnitude. Variations in temperature can indicate whether or not there is food present in the stomach. For example, an increase in temperature can indicate that food is being digested. FIG. 7 illustrates one exemplary embodiment of a triggering mechanism that includes a temperature sensor 700 for use with an implantable sensor of a restriction system. For purposes of illustration, the triggering mechanism is shown in association with the pressure measurement system described above with reference to FIG. 5. However, one skilled in the art will appreciate that the triggering mechanism can be used in conjunction with any type of sensor and its application should not be limited exclusively to pressure sensors or pressure measurement systems. As shown in FIGS. 7A-7C, the triggering mechanism takes the form of a temperature sensor 700 that is operatively associated with the pressure sensor 84. One skilled in the art will appreciate that the temperature sensor 700 can be disposed at a variety of locations within the system including, for example, on the implantable sensor, the implantable port, or within the esophagus or stomach. Additionally, one or more temperature sensors can be employed. The triggering event can generally be a change in temperature of a selected magnitude, a selected occurrence, or exceeding a temperature set point. For example, in one exemplary embodiment, the triggering event can be a change in temperature that is greater than or equal to 3°F. Thus, in use, a change in temperature detected by the temperature sensor greater than or equal to 3°F can be effective to trigger the implantable sensor, thereby energizing the implantable sensor from the dormant power usage mode to a use configuration. In another embodiment, the triggering event can be reaching a predetermined temperature set point(s). In yet another embodiment, the triggering event can be a series of alternating temperature cycles such as hot/cold/hot. As with the pH sensor trigger described above, a variety of techniques can be used to energize the system. In general, the temperature sensor can send a signal to the implantable sensor to energize. In one exemplary embodiment, the signal can be sent by means of a direct connection between the temperature sensor and the implantable sensor via a wired connection through the stomach (FIG. 7A). In another embodiment, the temperature sensor can wirelessly communicate with the implantable sensor (FIG. 7B). In yet another embodiment, the pH sensor can transmit a signal to an external collection device that communicates the temperature to the implantable sensor (FIG. 7C). One skilled in the art will appreciate that several techniques can be used to communicate the signal from the temperature sensor to the implantable sensor.

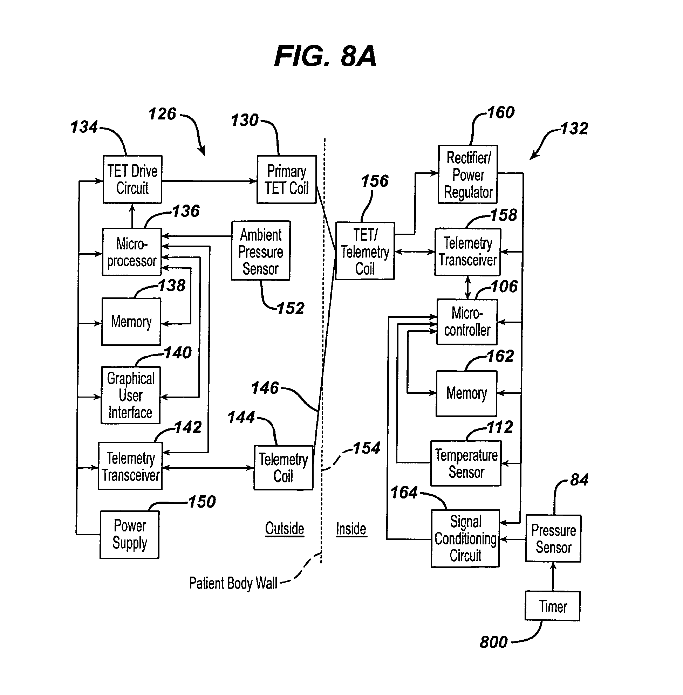

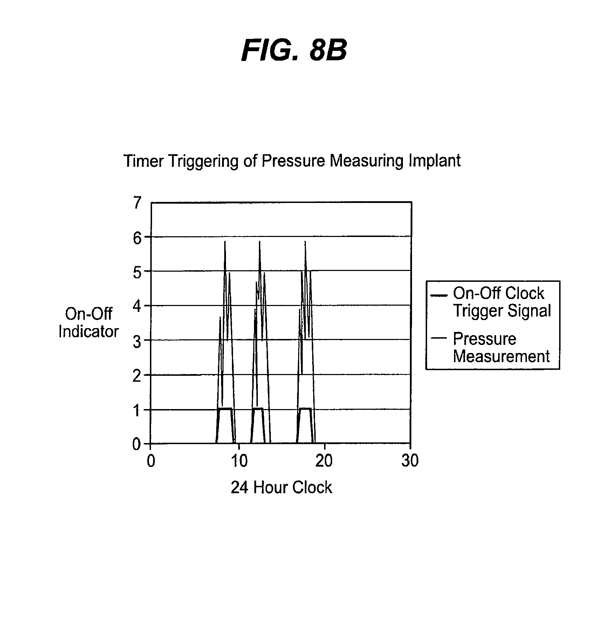

Another exemplary embodiment of a triggering mechanism is shown in FIG. 8A. As shown, the triggering mechanism includes a timer 800 that can be programmed to place the sensor in the use configuration at pre-determined intervals. For purposes of illustration, the triggering mechanism is shown in association with the pressure measurement system described above with respect to FIG. 5. However, one skilled in the art will appreciate that the triggering mechanism can be used in conjunction with any type of sensor and its application should not be limited exclusively to pressure sensors or pressure measurement systems. As shown in FIG. 8A, the timer 800 is operatively associated with the pressure sensor 84. One skilled in the art will appreciate that the timer 800 can be disposed at a variety of locations within the system including, for example, on the implantable sensor or the implantable port The timer 800 can be programmed to activate the implantable sensor at a variety of time intervals. In general, the timer 800 can be programmed to activate the sensor at time intervals that correspond to the mealtimes of the patient. Various configurations are available for programming the timer. For example, in one exemplary embodiment, the timer can be pre-programmed prior to implantation. In another embodiment, an external device can be used to initiate a program or adjust an existing program of a timer that is already implanted. Such a configuration can allow for adjustment of the time intervals as more is known about the patient's eating habits thereby enabling the system to adapt to the patient's habits by increasing or decreasing the sampling rate as necessary. FIG. 8B illustrates the effect of a programmed timer on a pressure sensor of a restriction system. As shown, pressure measurements are only recorded for the three programmed activation intervals, and the pressure sensor is in a non-operational state the remainder of the 24 hour period.

In yet another exemplary embodiment, the triggering mechanism can include a pressure sensor and the triggering event can be a change in pressure of a selected magnitude. The "pressure trigger" can have a variety of configurations and can take many forms, but is generally directed to detecting a change in pressure of a selected magnitude within the closed fluid circuit of the gastric restriction system. The physician is primarily concerned with pressures above a particular threshold, for example, 10mmHg for greater than 5 seconds. Thus, it is not necessary to actively sense and/or transmit data when the pressure within the closed fluid circuit is below this threshold. Further, when the pressure drops below the desired threshold after eating and peristalsis (i.e., the smooth muscle contractions that drive food distally through the esophagus, stomach, and intestines) are complete, the active components of the sensor can be shut-off entirely or can be defaulted to a low operating frequency to reduce power usage.

The "pressure trigger" can be an integral component of the pressure management system described above. For example, in one exemplary embodiment, the pressure sensor of the pressure management system can be configured to detect pressure changes of a selected magnitude at the low operating frequency of the dormant power usage mode. Thus, at the low operating frequency, only pressure changes of a selected magnitude are registered by the pressure sensor and the pressure sensor is not continuously sensing the fluid pressure within the system. Once the pressure sensor detects a change in pressure of a selected magnitude, for example, a change in baseline pressure that is greater than or equal to 10mmHg or a peak pressure greater than or equal to 60mmHg, the pressure sensor can be energized from the dormant state to the use configuration. One skilled in the art will appreciate that a variety of different values can be designated as the "selected magnitude," and the designation may vary from patient to patient. It can also be appreciated that the duration of the pressure magnitude may be factored in to determine if an authentic triggering event has occurred versus a transient event that does not require the system to be energized. A transient event can include, for example, a cough, a burp, and/or talking.

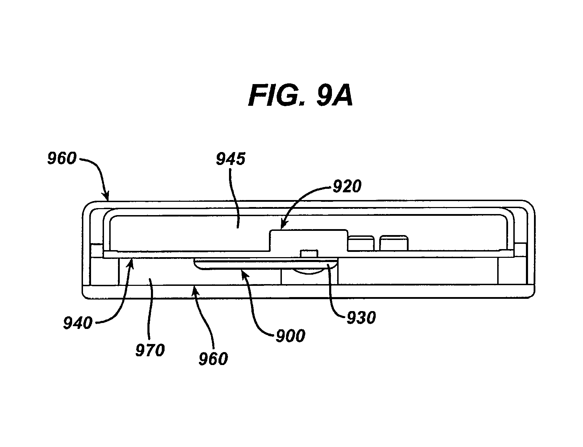

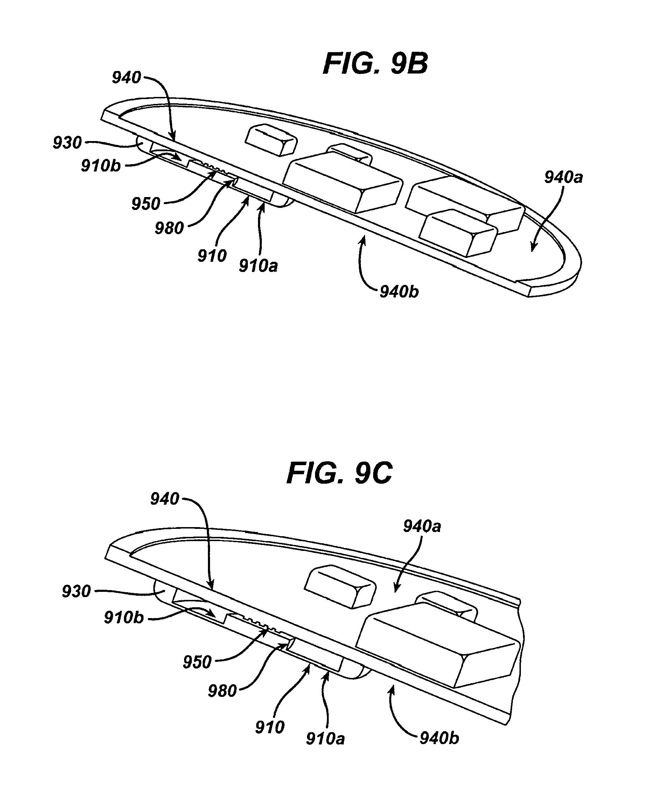

FIGS. 9A-9C illustrates another embodiment of a "pressure trigger." In this embodiment, the triggering mechanism 900 can include a flexible membrane 910 (FIGS. 9B and 9C) and the triggering event can be an increase in pressure within the restriction device that is effective to deflect the flexible membrane 910. The flexible membrane 910 can be at least partially conductive such that an increase in pressure can be effective to deflect the membrane 910 to complete an electrical circuit to energize the sensor 920 and place it in the use configuration. As described below, a variety of configurations are available for the flexible membrane 910.

In one exemplary embodiment illustrated in FIGS. 9A-9C, the flexible membrane 910 is part of a metal capsule 930 that is disposed on a surface of a PC board 940 that contains the sensor electronics 945. The PC board 940 can generally be formed of a glass or ceramic material thereby allowing the metal capsule 930 to be brazed to a surface of the board 940. One surface 940a of the PC board 940 can contain the sensor electronics 945, and another surface 940b of the PC board 940 can have interlaced finger traces 950 formed thereon. The metal capsule 930 can be brazed onto the surface 940b of the PC board 940 containing the finger traces 950 thereby hermetically sealing the finger traces 950 from the fluid of the closed circuit. As shown in FIG. 9A, the metal capsule 930, PC board 940, and pressure sensor 920 can all be hermetically sealed within an outer capsule 960 to improve the long-term functioning of the device.

The circumference of the metal capsule 930 can be very stiff relative to the pressures of the fluid, and the flexible membrane 910 can extend across the stiff outer perimeter of the capsule 930 such that the flexible membrane 910 is allowed to deflect. A first surface 910a of the flexible membrane 910 can be in contact with the fluid 970 in the closed circuit. A second surface 910b of the flexible membrane 910 opposite the first surface 910a and not in contact with the fluid 970 in the system can have a conductive "pill" 980 disposed thereon. As shown in FIGS. 9B and 9C, the conductive "pill" 980 is disposed directly on the flexible membrane 910. In another exemplary embodiment, the conductive "pill" can be mounted on a flexible elastomeric element that is disposed between the flexible membrane and the conductive "pill." Such a configuration can electrically isolate the metal structure of capsule from the conductive "pill" as well as allow the membrane to flex in an arcuate fashion while the "pill" contacts at least two separate fingers simultaneously.

The conductive "pill" 980 can be configured to engage the finger traces 950 formed on the PC board 940. Under elevated pressure conditions, pressure from the fluid 970 within the closed circuit can deflect the flexible membrane 910 and push the conductive "pill" 980 into contact with the finger traces 950. The flexible membrane 910 can be configured to deflect at a pre-determined pressure. For example, in one embodiment a change in fluid pressure of 10mmHg can be effective to deflect the membrane 910 to cause the conductive "pill" 980 to engage the finger traces 950. In another exemplary embodiment, the flexible membrane 910 can be configured to deflect when the fluid pressure is greater than or equal to a pre-determined threshold value, such as 70 mmHg. The conductive "pill" 980 can make an electrical connection across the finger traces 950 thereby triggering the onboard circuitry contained in the PC board 940 to activate the sensor 920. When the pressure drops below the set point for a predetermined amount of time and the "pill" 980 is no longer in contact with the traces 950, the sensor 920 can return to the default dormant power usage mode.

In each of the embodiments described above, the triggering mechanism is configured to automatically activate the implantable sensor in response to a physiological change in the patient or other change within the restriction system. The triggering mechanisms described below do not automatically activate the sensor in response to a system change but, instead, enable the patient or physician to activate the sensor at appropriate intervals, such as mealtimes.

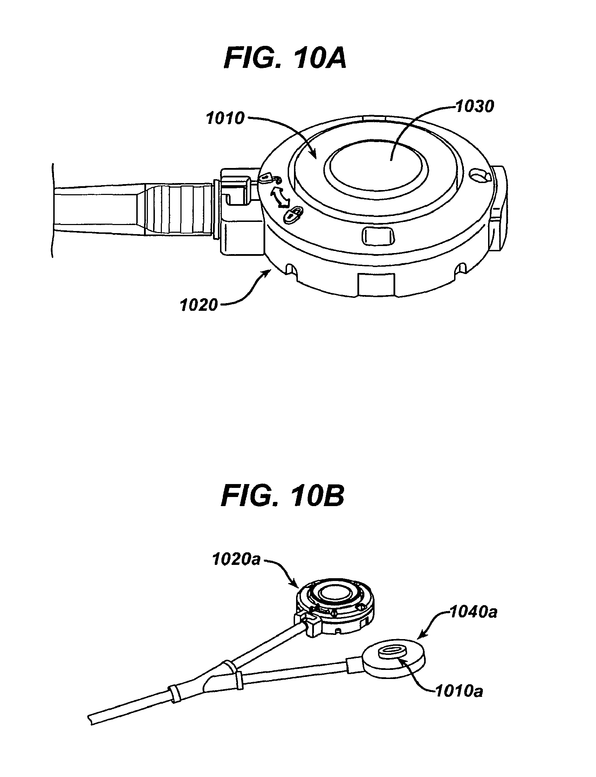

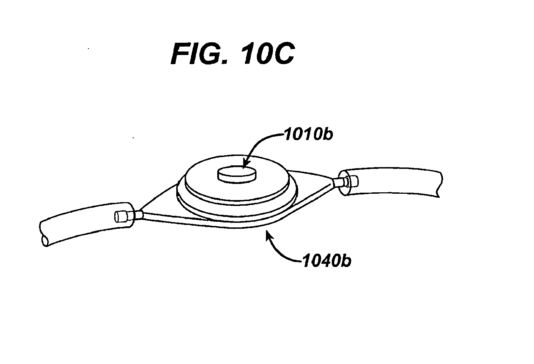

FIGS. 10A-10C illustrate one exemplary embodiment of a triggering mechanism that allows the patient or physician to activate the sensor when desired. The triggering mechanism can generally include an actuator and the triggering event includes actuation of the actuator. The actuator can have a variety of configurations and can take several shapes and sizes. As shown in FIG. 10A, the actuator 1010 takes the form of a manual button that is substantially circular in shape and is disposed on the implantable port 1020 surrounding the injection port 1030. Although the actuator is shown and described as being generally circular in shape, one skilled in the art will appreciate that the actuator can take any shape. Additionally, the actuator need not be disposed on the implantable port. For example, as shown in FIG. 10B, the actuator 1010a can be disposed on a sensor and/or transmitter 1040s that is tied off of the line from the port 1020s to the band. In another exemplary embodiment, shown in FIG. 10C, the actuator 1010b can be disposed on a sensor and/or transmitter 1040b that is in-line between the band and the injection port.

The actuator can be palpable through the skin in a manner similar to how the physician palpates for the port for needle insertion during band adjustments. Thus, in use, the patient and/or physician can locate the actuator through the skin and apply a force to the actuator to activate the sensor. In another embodiment, the actuator can be activated by magnetic force. For example, the actuator can include a ferromagnetic metallic component that is biased posteriorly with a spring force. In use, the patient or physician can pass a magnet over the actuator to overcome the spring force and allow the metallic component to move anterior and complete an electrical circuit thereby activating the sensor.

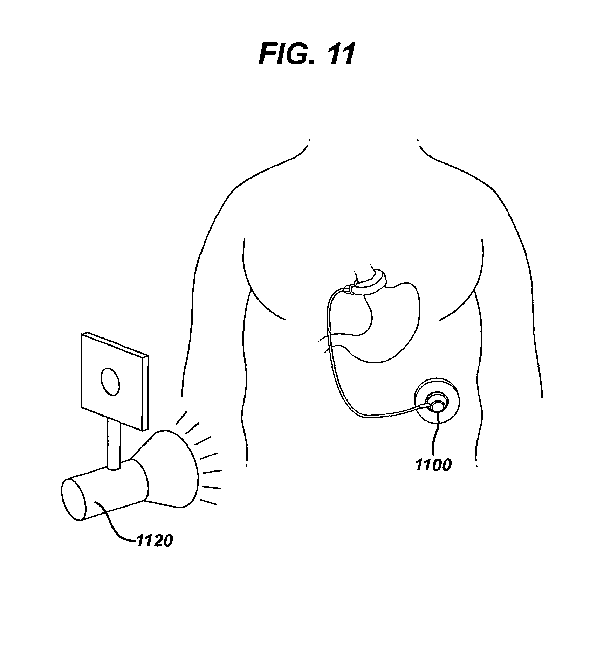

Another exemplary embodiment of a manually activated triggering mechanism is shown in FIG. 11. The triggering mechanism can include an implantable photoreceptor 1100 and the triggering event can include subjecting the implantable photoreceptor to a light source. For example, in one exemplary embodiment the patient can ingest an LED equipped pill to trigger the implantable photoreceptor 1110. In another embodiment, an external device 1120 can be used to trigger the photoreceptor 1100. In general, light can penetrate several layers of tissue. Infrared light, in particular, has been shown to be especially advantageous for locating veins and arteries because infrared light easily passes through a patient's body except where it is absorbed and partially blocked by blood thereby highlighting the patient's veins and arteries. Thus, because infrared light can easily penetrate several layers of tissue, a patient or physician can use an external infrared light source 1120 to subject a photoreceptor 1100 disposed in the restriction system to infrared light to thereby activate the implantable sensor. The implantable photoreceptor can be disposed on the implantable port, the implantable sensor, or at another location within the restriction system such as on or in close proximity to the gastric wall. One skilled in the art will appreciate that any wavelength capable of penetrating tissue can be used to engage the photoreceptor. Additionally, the external device can project a single continuous beam of light or a specific pattern of light. Projecting a pattern of light, such as a flashing beam, can prevent the photoreceptor from being inadvertently triggered by an incidental light source. One skilled in the art will recognize that one or more triggering patterns can be established.

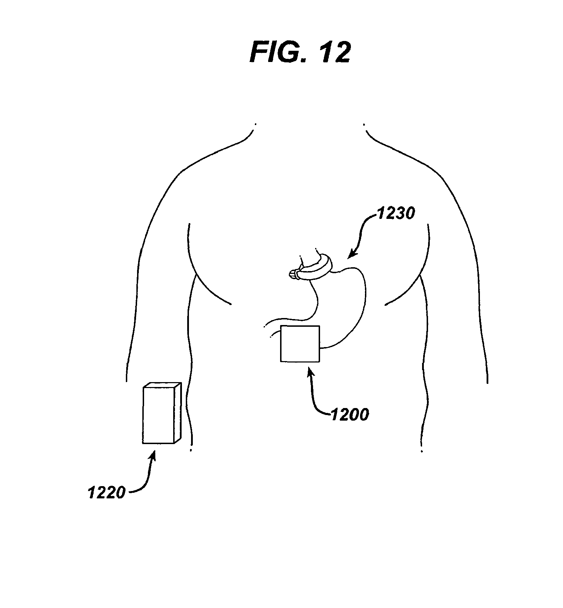

FIG. 12 illustrates another exemplary embodiment of a manually activated triggering mechanism. In this embodiment, the triggering mechanism can include an implantable accelerometer 1200 and the triggering event can include transmitting vibratory energy within a selected frequency range transdermally to the implantable accelerometer with an external actuator 1220. In general, an accelerometer is an electromechanical device that can measure acceleration forces such as, for example, dynamic forces caused by moving or vibrating the accelerometer. Thus, the patient or physician can use an external device 1220, such as a vibrating wand, to transmit vibratory energy through the skin to an implantable accelerometer 1200 disposed in the restriction system 1230 to thereby activate the implantable sensor. The implantable accelerometer can be disposed on the implantable port, the implantable sensor, or at another suitable location within the restriction system. Various types of accelerometers can be incorporated into the triggering mechanism including, for example, capacitive, piezoelectric, piezoresistive, Hall-effect, magnetoresistive, and heat transfer accelerometers. One skilled in the art will appreciate that vibratory energy within virtually any frequency range (and optionally for a selected period of time) can be selected to excite the accelerometer and thereby activate the sensor. For example, in one exemplary embodiment, the external device can transmit vibratory energy having a frequency that is greater than or equal to 1 to 3 Hz. As with the photoreceptor embodiment, the vibratory energy can be a single frequency or a series of pre-selected alternating frequencies (e.g., Morse Code) to prevent the accelerometer from inadvertently triggering from everyday activity.

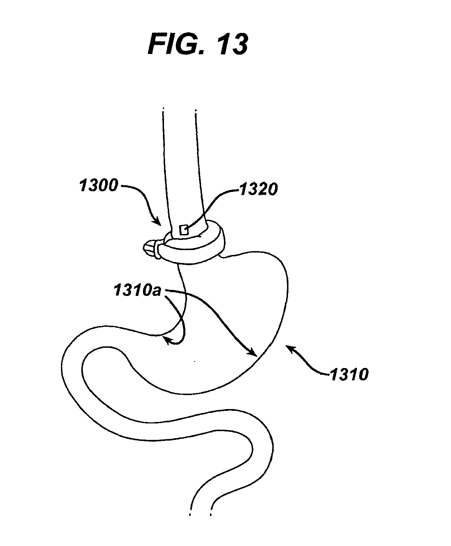

Yet another embodiment of a manually activated triggering mechanism is shown in FIG. 13. In this embodiment, the triggering means can be transmitted from within the stomach. As shown in FIG. 13, the triggering mechanism includes a sensor element 1300 that is disposed in close proximity to the surface 1310a of the stomach 1310. The triggering event can include passing a triggering element 1320 through the stomach to thereby trigger the sensor element 1300 and activate the implantable sensor. For example, in one exemplary embodiment, the sensor element can be a temperature sensor that is configured to detect the internal temperature of the stomach. The triggering event can include changing the internal temperature of the stomach by having the patient ingest an alternating series of hot and cold liquids in a predetermined pattern. Such a configuration can prevent the device from being inadvertently triggered when it is not mealtime and the patient simply ingests a hot or cold drink. In another embodiment, the sensor element can be a magnetic sensor that is configured to detect magnetic fields within the stomach. The triggering event can include generating a magnetic field within the stomach. The magnetic field can be generated by having the patient ingest a magnetic element such as a small magnetic pill or a magnetic powder that is mixed with food or drink. Thus, as the magnetic pill or powder passes through the patient's stomach, the magnetic field associated with the magnetic element is detected by the magnetic sensor thereby activating the implantable sensor.

Regardless of whether the implantable sensor is automatically energized in response to a change in operating parameter of the system or physiological characteristic of the patient or manually energized by the patient or physician, energizing the sensor is effective to place the sensor in the use configuration. In the use configuration, the sensor can collect data related to the operation of the restriction device and transmit the collected data to an external device. The physician can then use the collected data to make adjustments to the restriction system to optimize the performance of the system.

A person skilled in the art will appreciate that the present invention is described in the context of a pressure sensor being selectively activated from a dormant power usage mode to a use configuration. However, it is understood that a variety of other sensors (i.e., for detecting other physiological and non-physiological parameters) can be used in addition to or as an alternative to a pressure sensor. The present invention is also applicable to the triggering of such sensors to a use mode.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A restriction system for forming a restriction in a patient, comprising:
an implantable restriction device, the restriction device being adjustable and configured to form a restriction in a patient; and
an implantable sensor in communication with the restriction device, the sensor being defaulted to a dormant power usage mode and having a triggering mechanism configured to place the sensor in a use configuration upon the occurrence of a triggering event.

2. The restriction system of claim 1, further comprising an implantable port in fluid communication with the implantable restriction device and configured to receive fluid from a fluid source external to the patient.

3. The restriction system of claim 2, wherein the implantable sensor is integrated with the port.

4. The restriction system of claim 2, wherein the triggering mechanism is formed on either the implantable sensor or the implantable port.

5. The restriction system of claim 1, wherein the dormant power usage mode has an operating frequency less than or equal to about 1 Hz.

6. The restriction system of claim 1, wherein the use configuration has an operating frequency in the range of about 2 to 20 Hz.

7. The restriction system of claim 1, wherein the triggering mechanism includes at least one gastric pH sensor and the triggering event is a change in gastric pH of a selected magnitude detected by the gastric pH sensor.

8. The restriction system of claim 1, wherein the triggering mechanism includes at least one pressure sensor and the triggering event is a change in pressure of a selected magnitude detected by the pressure sensor.

9. The restriction system of claim 1, wherein the trigging mechanism includes a flexible membrane and the triggering event is an increase in pressure within the restriction device that is effective to deflect the flexible membrane.

10. The restriction system of claim 9, wherein the flexible membrane is at least partially conductive and deflecting the membrane is effective to complete an electrical circuit to energize the sensor and place it in the use configuration.

11. The restriction system of claim 1, wherein the triggering mechanism is an actuator and the triggering event includes actuation of the actuator.

12. The restriction system of claim 1, wherein the triggering mechanism includes a magnetic sensor and the triggering event includes generating at least one magnetic field thereby engaging the triggering mechanism.

13. The restriction system of claim 1, wherein the triggering mechanism includes a photoreceptor and the triggering event includes subjecting the photoreceptor to a light source using an external device.

14. A method of effecting gastric restriction, comprising:
providing an implantable restriction system for forming a restriction in a patient, the system including an adjustable restriction device configured to form a restriction in a patient, and a sensor in communication with the restriction device and being selectively configured between a dormant power usage mode and a use mode;
triggering the sensor in response to a selected stimulus to energize the sensor from the dormant power usage mode to the use mode;
collecting data related to the operation of the adjustable restriction device via the sensor when the sensor is in the use mode; and
transmitting the data collected by the sensor to an external device when the sensor is in the use mode.

15. A method of energizing an implantable sensor, comprising:
providing an implantable sensor in communication with an adjustable gastric restriction device, the sensor being selectively configured between a dormant power usage mode and a use mode for collecting and transmitting data related to the operation of the adjustable gastric restriction device; and
triggering the sensor in response to a selected stimulus to energize the sensor from the dormant power usage mode to the use mode.
